# EUROPÄISCHE PATENTSCHRIFT

(11) **EP 0 501 388 B1**
(45) Veröffentlichungstag und Bekanntmachung des Hinweises auf die Patenterteilung: **28.06.1995**
(21) Anmeldenummer: 92103083.9
(22) Anmeldetag: 24.02.1992
(51) Int. Cl.: A61M 11/00, B05B 11/00

(54) **Sprühflasche**
Spray bottle
Flacon pulvérisateur

(30) Priorität: 01.03.1991 DE 4106575
(43) Veröffentlichungstag der Anmeldung: 02.09.1992
(73) Patentinhaber: Perfect-Valois Ventil GmbH, 44319 Dortmund (DE)
(72) Erfinder: Schmitz, Detlef, W-4670 Lünen (DE)
(74) Vertreter: Strehl Schübel-Hopf Groening & Partner

(56) Entgegenhaltungen:
- DE-A- 2 040 635
- FR-A- 1 028 776
- US-A- 2 676 845
- US-A- 3 176 883

## Beschreibung

Sprühvorrichtung für eine Sprühflasche mit flexibler, von Hand zusammendrückbarer Wandung zum Einbringen eines flüssigen Medikamentes in eine Öffnung eines menschlichen oder tierischen Körpers, bestehend aus einem hohlzylindrischen Stopfen, der in die Öffnung der Flasche einsetzbar ist, und einer Sprühkappe, die mit einem äußeren Ringflansch auf dem Stirnrand des die Öffnung umgebenden Flaschenhalses aufliegt und deren elastische Kappenwand sich nach oben stumpf kegelförmig verjüngt und in einer Kopfwand mit einer Sprühöffnung endet;
einem zylindrischen Stutzen an der Unterseite der Kopfwand, in den ein Steigrohr eingesetzt ist, das sich bis zum Boden der Sprühflasche erstreckt und mit der Sprühöffnung in Flüssigkeitsverbindung steht;
einem Mischluftkanal, der sich zwischen dem zylindrischen Stutzen und dem Steigrohr in einen Mischraum zwischen der Stirnseite des Steigrohres und der Sprühöffnung erstreckt;
sowie einem Belüftungskanal, dessen radialer Teil in dem Ringflansch der Sprühkappe vorgesehen ist und der in Richtung des Innenraums der Sprühflasche abgewinkelt sowie mit einem Absperrventil versehen ist.

Eine Sprühflasche dieser Gattung ist aus der US-PS 3,176,883 bekannt. Die Sprühvorrichtung dieser bekannten Sprühflasche ist mehrteilig und daher sowohl hinsichtlich der zur Herstellung notwendigen Werkzeuge als auch der Montage außerordentlich aufwendig. Der Belüftungkanal erstreckt sich durch zwei unterschiedliche Bestandteile der Sprühvorrichtung und ist durch eine elastische, an der Unterseite eines Ventilgehäuses in der Mitte gehaltene Ventilscheibe verschlossen. Die Betätigung dieser Ventilscheibe setzt eine bestimmte Druckdifferenz zwischen dem Druck der äußeren Atmosphäre und im Innenraum der Sprühfasche voraus. Außerdem ist der Querschnitt des Belüftungskanals so klein bemessen, daß einer schnellen Belüftung der Flasche nach deren Betätigung durch Zusammendrücken ihrer elastischen Seitenwände nicht nur die elastische Ventilscheibe, sondern auch die Drosselwirkung des Belüftungskanals entgegensteht.

Der Erfindung liegt die Aufgabe zugrunde, stets eine vollständige und schnelle Belüftung der Sprühflasche mittels einer Sprühvorrichtung zu ermöglichen, durch die sichergestellt ist, daß die Sprühflasche in kürzester Zeit mehrfach hintereinander zur Abgabe des in der Flasche enthaltenen Medikamentes in Form eines in die betreffende Körperöffnung abgegebenen Sprühstrahls betätigt werden kann. Daneben soll ein einfacher Aufbau eine wirtschaftliche Herstellung und Montage gewährleisten.

Die Erfindung löst diese Aufgabe durch die Kombination folgender Merkmale:
- Die Sprühvorrichtung ist einstückig ausgebildet;
- der radiale Teil des Belüftungskanals reicht bis an das Steigrohr, wobei der in das Innere der Sprühflasche abgewinkelte Teil des Belüftungskanals von der Innenseite des hohlzylindrischen Stopfens sowie durch eine Wand begrenzt ist, die sich parallel zum Steigrohr erstreckt;
- das obere Ende des abgewinkelten Teils des Belüftungskanals ist mit einem Ventilsitz für eine Ventilkugel versehen;
- das untere Ende des abgewinkelten Teils des Belüftungskanals weist Tragarme für die Ventilkugel auf, wobei die Tragarme in Umfangsabständen in den freien Querschnitt des abgewinkelten Kanalabschnitts hineinragen und auch bei aufliegender Ventilkugel eine Belüftung der Sprühflasche ermöglichen.

Die einstückige Ausbildung der Sprühvorrichtung ermöglicht eine Einsparung der Werkzeug- und damit der Herstellungskosten der Sprühvorrichtung und gleichzeitig eine einfache und schnelle Montage derselben. Ferner ist der Materialeinsatz für jede Sprühvorrichtung äußerst gering. Dadurch, daß der radiale Teil des Belüftungskanals bis an das Steigrohr heranreicht, wird der lichte radiale Abstand zwischen der Innenseite des hohlzylindrischen Stopfens und der an das Steigrohr angrenzenden Wand ausgenutzt, um dem in das Innere abgewinkelten Teil des Belüftungskanals einen möglichst großen Querschnitt für die ständige Belüftung des Innenraums der Sprühflasche zu geben. Die Anordnung des Absperrventils in dem abgewinkelten Teil des Belüftungskanals mit dem Ventilsitz am oberen Ende für die Ventilkugel ermöglicht eine schnelle Reaktionsfähigkeit der Ventilkugel auf einen in der Sprühflasche herrschenden Überdruck durch Zusammenpressen ihrer elastischen Wandung im Schließsinne des Ventils und gleichzeitig ein sofortiges Öffnen des Ventils nicht nur durch Druckdifferenz, sondern auch durch die auf die Ventilkugel einwirkende Schwerkraft, derart, daß die Ventilkugel durch ihre Auflage auf den Tragarmen den abgewinkelten Kanalabschnitt teilweise für eine dauernde Belüftung der Sprühflasche freihält, solange diese nicht betätigt wird. Außerdem stellt diese ständige Belüftung der Sprühflasche im nichtbetätigten Zustand einen vollständigen und schnellen Druckausgleich zwischen der Außenatmosphäre und dem Innenraum der Sprühflasche sicher. Dies ist insbesondere von Bedeutung bei der Bekämpfung von anfallartig auftretenden Krankheitserscheinungen, wie sie bei Asthma, Herzerkrankungen, Zuckerkrankheiten usw. auftreten, bei denen unter Umständen ein sofortiges und mehrfaches Einsprühen des Medikamentes in die betreffende Körperöffnung notwendig ist. In diesem Fall ist die sofortige und vollständige Belüftung der Sprühflasche nach ihrer Betätigung auch von psychologischer Bedeutung für den betreffenden Patienten, weil die sofortige Rückstellung der zusammengedrückten Flaschenwandung ihm das erwünschte Sicherheitsgefühl bezüglich der sofortigen, wiederholbaren Einsatzbereitschaft der Sprühflasche vermittelt.

Die Erfindung ist nachstehend anhand der schematischen Zeichnung eines Ausführungsbeispiels einer Sprühflasche näher erläutert. Es zeigen:
- Fig. 1: einen mittleren Längsschnitt durch eine Sprühflasche in Ruhestellung;
- Fig. 2: die Sprühflasche gemäß Fig. 1 in Betätigungsstellung, und
- Fig. 3: die Sprühflasche gemäß Fig. 1 oder 2 in Ansaugstellung.

In den Figuren ist eine flexible, von Hand zusammendrückbare Sprühflasche 10 aus Kunststoff zum Einbringen eines vorzugsweise flüssigen Medikamentes 11 in eine Öffnung, vorzugsweise Nasenkanal, eines menschlichen oder tierischen Körpers gezeigt. Eine Sprühkappe 12 ist mit einem etwa zylindrischen Stopfen 13, der sich nach unten geringfügig verjüngt, in einen Hals 14 der Sprühflasche 10 abdichtend eingesetzt, der die Flaschenöffnung bildet. Die Sprühkappe 12 liegt mit einem äußeren Ringflansch 15 auf dem oberen Stirnrand 16 des Flaschenhalses 14 auf. Eine elastische Kappenwand 32 mit einem der Körperöffnung angepaßten Durchmesser erstreckt sich von dem Ringflansch 15 unter stumpfkegelartiger Verjüngung nach oben und bildet eine Kopfwand 17. Ein zylindrischer Stutzen 18 mit einer Ausnehmung 19 steht von der Unterseite der Kopfwand 17 nach unten vor, die mit einer Sprühöffnung 20 versehen ist. Die Sprühöffnung 20 ist koaxial zur Ausnehmung 19 angeordnet. In die Ausnehmung 19 ist ein Steigrohr 21 eingesetzt, das sich bis zum Flaschenboden 22 erstreckt und mit der Sprühöffnung 20 in Verbindung steht. Ferner ist ein Belüftungskanal 23 für den Luftraum 24 innerhalb der Sprühflasche 10 vorgesehen, dem ein Absperrventil 33 zugeordnet ist, das ausschließlich bei Überdruck in der Sprühflasche 10 geschlossen ist.

Der Belüftungskanal 23 erstreckt sich in dem Ringflansch 15 der Sprühkappe 12 radial zur Hauptachse derselben in einen Ringraum 25 zwischen der Innenseite der Kappenwand 32 und dem Steigrohr 21 bis an das Steigrohr 21. Im Bereich des Ringraums 25 ist der Belüftungskanal 23 nach unten zum Inneren der Sprühflasche 10 hin abgewinkelt und wird dabei von der Innenseite des etwa zylindrischen Stopfens 13 der Sprühkappe 12 an der Außenseite sowie durch eine Wand 26 begrenzt, die sich parallel entlang dem Steigrohr 21 erstreckt. Das obere Ende des nach unten abgewinkelten Belüftungskanals 23 ist mit einem Ventilsitz 27 für einen Ventilkörper 28 versehen. Der Ventilkörper 28 besteht im vorliegenden Ausführungsbeispiel aus einer Kugel. Diese Ventilkugel kann aus Metall sein. Die Kugeloberfläche kann mit einer Kunststoffschicht überzogen sein, falls ungünstige chemische Reaktionen des in der Sprühflasche enthaltenen Wirkstoffs mit dem Metall zu befürchten sind. In diesem Fall kann die Ventilkugel ggf. auch vollständig aus Kunststoff hergestellt sein.

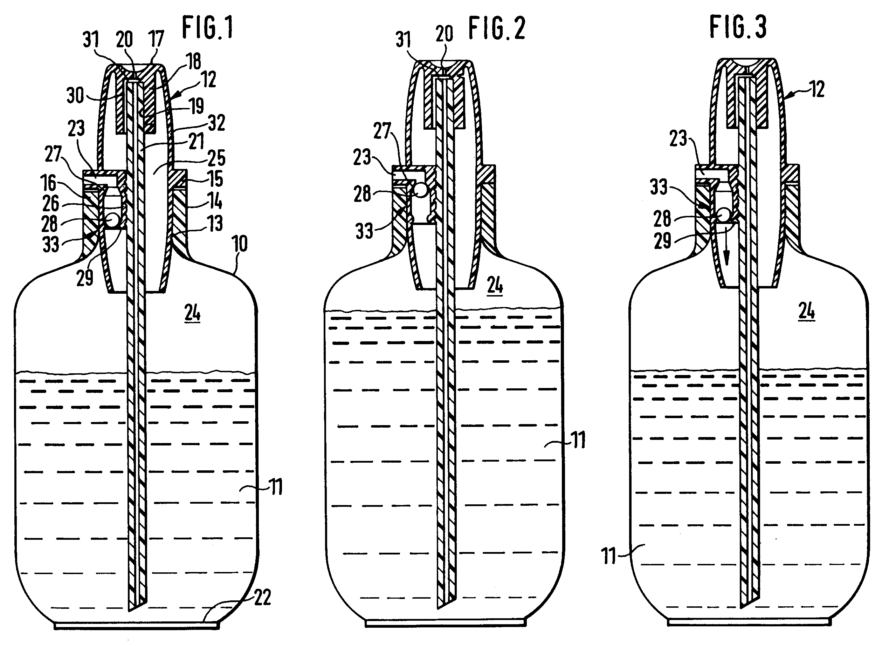

Das untere Ende des nach unten abgewinkelten Abschnitts des Belüftungskanals 23 ist mit einer die Belüftung des Innenraums 24 der Sprühflasche 10 ermöglichenden Haltevorrichtung für den Ventilkörper 28 versehen. Diese Haltevorrichtung besteht aus in den freien Querschnitt des Belüftungskanals 23 radial nach innen vorspringenden Tragarmen 29, die in Umfangsabständen voneinander verteilt angeordnet sind und zwischen sich einen Querschnitt auch dann freilassen, wenn der Ventilkörper 28 auf diesen Tragarmen 29 ruht. Infolgedessen ist der Innenraum 24 der Sprühflasche 10 in Fig. 1 belüftet, da die Außenluft über den Belüftungskanal 23 und die von dem Ventilkörper 28 in der Öffnungsstellung freigelassenen Durchtrittsquerschnitte sowie über den Ringraum 25 zwischen dem Stopfen 13 und dem Steigrohr 21 mit dem Innenraum 24 dauernd in Verbindung steht.

Ein Mischluftkanal 30 erstreckt sich zwischen dem Stutzen 18 und dem Steigrohr 21 bis zu einem Mischraum 31, der zwischen der Mündung des Steigrohres 21 und der Sprühöffnung 20 angeordnet ist und dazu dient, die in der Sprühflasche 10 enthaltene Flüssigkeit 11 mit der Luft zu vermischen und auf diese Weise eine feine Verteilung der Sprühflüssigkeit in dem Sprühstrahl zu erreichen, der die Sprühöffnung 20 verläßt.

Im Betätigungszustand der Sprühflasche 10 in Fig. 2 wird das Medikament durch den durch das Zusammenpressen der flexiblen Flaschenwände hervorgerufenen Überdruck durch ein Steigrohr 21 hindurch von der freien Atmosphäre angesaugt und im Mischraum 31 vorher mit der im Innenraum 24 komprimierten Luft vermischt. Wie ersichtlich, wird durch die komprimierte Luft im Innenraum 24 der Sprühflasche 10 die Ventilkugel 28 auf ihren oberen Ventilsitz 27 gepreßt, wodurch ein Austritt der in der Sprühflasche enthaltenen Luft durch den Belüftungskanal 23 ins Freie verhindert wird.

Sobald sich die flexiblen Flaschenwände nach ihrer Betätigung wieder in ihre Ausgangslage zurückbewegen, wird der Ventilkörper 28 in seine untere Belüftungsstellung gemäß Fig. 3 zurückbewegt, so daß die Außenluft schnell durch den Belüftungskanal 23 hindurch und vorbei an dem Ventilkörper 28 in der in Fig. 3 gezeigten Stellung den Innenraum 24 der Sprühflasche lüften und einen Druckausgleich herstellen kann, durch den die Flaschenwände schnell ihre ursprüngliche Lage wieder einnehmen können und die Sprühflasche 10 für einen nächsten Sprühvorgang bereit ist.

Es ist ersichtlich, daß der einfache Aufbau der Sprühkappe 12 und des darin angeordneten Belüftungsventils nur geringfügige Änderungen bekannter Sprühkappen erfordert und somit eine wirksame Belüftung einer derartigen, für Nasenspray bevorzugten Sprühflasche 10 ermöglicht, ohne daß bei der Belüftung der Sprühflasche 10 Körpersekret durch die Sprühöffnung 20 in die Sprühflasche angesaugt wird.

## Patentansprüche

1. Sprühvorrichtung für eine Sprühflasche (10) mit flexibler, von Hand zusammendrückbarer Wandung zum Einbringen eines flüssigen Medikamentes (11) in eine Öffnung eines menschlichen oder tierischen Körpers, bestehend aus einem hohlzylindrischen Stopfen (13), der in die Öffnung der Sprühflasche (10,14) einsetzbar ist, und einer Sprühkappe (12), die mit einem äußeren Ringflansch (15) auf dem Stirnrand (16) des die Öffnung umgebenden Flaschenhalses (14) aufliegt und deren elastische Kappenwand (32) sich nach oben stumpf kegelförmig verjüngt und in einer Kopfwand (17) mit einer Sprühöffnung (20) endet,
einem hohlzylindrischen Stutzen (18) an der Unterseite der Kopfwand (17), in den ein Steigrohr (21) eingesetzt ist, das sich bis zum Boden (22) der Sprühflasche (10) erstreckt und mit der Sprühöffnung (20) in Flüssigkeitsverbindung steht,
einem Mischluftkanal (30), der sich zwischen dem zylindrischen Stutzen (18) und dem Steigrohr (21) in einen Mischraum (31) zwischen der Stirnseite des Steigrohres (21) und der Sprühöffnung (20) erstreckt;
sowie
einem Belüftungskanal (23), dessen radialer Teil in dem Ringflansch (15) der Sprühkappe (12) vorgesehen ist und der in Richtung des Innenraums (24) der Sprühflasche (10) abgewinkelt sowie mit einem Absperrventil (33) versehen ist,
gekennzeichnet durch die Kombination folgender Merkmale:
Die Sprühvorrichtung ist einstückig ausgebildet;
der radiale Teil des Belüftungskanals (23) reicht bis an das Steigrohr (21), wobei der in das Innere der Sprühflasche (10) abgewinkelte Teil des Belüftungskanals (23) von der Innenseite des hohlzylindrischen Stopfens (13) sowie durch eine Wand (26) begrenzt ist, die sich parallel zum Steigrohr (21) erstreckt,
das obere Ende des abgewinkelten Teils des Belüftungskanals (23) ist mit einem Ventilsitz (27) für eine Ventilkugel (28) versehen;
das untere Ende des abgewinkelten Teils des Belüftungskanals (23) weist Tragarme (29) für die Ventilkugel (28) auf, wobei die Tragarme (29) in Umfangsabständen in den freien Querschnitt des abgewinkelten Kanalabschnitts hineinragen und auch bei aufliegender Ventilkugel (28) eine Belüftung der Sprühflasche (10) ermöglichen.

## Claims

1. Spray device for a spray bottle (10) with a flexible, manually compressible wall for applying a liquid medicament (11) into an opening of a human or animal body comprising a hollow cylindrical plug (13), which may be inserted into the opening of the spray bottle (10,14), and a spray cap (12), which rests with an external annular flange (15) on the end edge (16) of the bottle throat (14) surrounding the opening and whose elastic cap wall (32) tapers upwardly frusto-conically and terminates in a top wall (17) with a spray opening (20),
a hollow cylindrical socket (18) on the underside of the top wall (17) in which a feed pipe (21) is inserted which extends to the base (22) of the spray bottle (10) and is in liquid communication with the spray opening (20), a mixing air passage (30) which extends between the cylindrical socket (18) and the feed pipe (21) into a mixing space (31) between the end face of the feed pipe (21) and the spray opening (20);
and an aeration passage (23), whose radial portion is provided in the annular flange (15) of the spray cap (12) and which is bent in the direction of the internal space (24) of the spray bottle (10) and is provided with a shut off valve (33),
characterised by the combination of the following features:
The spray device is of one-piece construction;
The radial portion of the aeration passage (23) extends to the feed pipe (21), whereby the portion of the aeration passage (23) bent into the interior of the spray bottle (10) is defined by the inner surface of the hollow cylindrical plug (13) and by a wall (26) which extends parallel to the feed pipe (21),
The upper end of the bent portion of the aeration passage (23) is provided with a valve seat (27) for a valve ball (28);
The lower end of the bent portion of the aeration passage (23) has support arms (29) for the valve ball (28), whereby the support arms (29) project at circumferential spacings into the free cross-section of the bent passage section and render aeration of the spray bottle (10) possible even when the valve ball (28) rests on them.

## Revendications

1. Dispositif de pulvérisation pour un flacon pulvérisateur (10), avec une paroi flexible, pouvant être comprimée à la main, pour introduire un médicament (1) liquide dans une ouverture d'un corps humain ou animal, composé d'un bouchon (13) cylindrique creux pouvant être inséré dans l'ouverture du flacon pulvérisateur (10, 14) et d'un capuchon pulvérisateur (12), reposant, par une bride annulaire (15) extérieure, sur la bordure frontale (16) du col de flacon (14) entourant l'ouverture et dont la paroi de capuchon (32) élastique va en s'effilant de façon tronconique vers le haut et s'achève en une paroi de tête (17) avec une ouverture de pulvérisation (24), avec une tubulure (18) cylindrique creuse en face inférieure de la paroi de tête (17), dans laquelle est intégré un tube montant (21) qui s'étend jusqu'au fond (22) du flacon pulvérisateur (10) et est relié hydrauliquement à l'ouverture de pulvérisation (20), avec un canal d'air de mélange (30) s'étendant entre la tubulure cylindrique (18) et le tube montant (21), allant dans un espace de mélange (31) situé entre la face frontale du tube montant (21) et l'ouverture de pulvérisation (20); ainsi qu'un canal d'aération (23), dont la partie radiale est prévue dans la bride annulaire (15) du capuchon pulvérisateur (12) et qui est coudé en direction de l'espace intérieur (24) du flacon pulvérisateur (10) et est pourvu d'une soupape d'isolement (33),
caractérisé par la combinaison des caractéristiques ci-après :
le dispositif pulvérisateur est réalisé d'une seule pièce ;
la partie radiale du canal d'aération (23) arrive jusqu'au tube montant (21), la partie coudée, à l'intérieur du flacon pulvérisateur (10) du canal d'aération (23), étant délimitée par la face intérieure du bouchon (13) cylindrique creux, ainsi que par une paroi (26) qui s'étend parallèlement au tube montant (21),
l'extrémité supérieure de la partie coudée du canal d'aération (23) est pourvue d'un siège de soupape (27) pour une bille d'opercule (28);
l'extrémité inférieure de la partie coudée du canal d'aération (23) présente des bras-support (29) pour les billes d'opercule (28), les bras-support (29) pénétrant, selon des espacements périphériques, dans la section transversale libre de la section de canal coudée et permettant également une aération du flacon pulvérisateur (10) lorsque la bille d'obturateur (28) est en appui.
